# EUROPEAN PATENT SPECIFICATION

(11) **EP 0 895 085 B1**
(45) Date of publication and mention of the grant of the patent: **05.02.2003**
(21) Application number: 98113378.8
(22) Date of filing: 17.07.1998
(51) Int. Cl.: G01N 33/574, G01N 33/543

(54) **Immunoassay for determination of PSA-ACT**
Immunoassay zur Bestimmung von PSA-ACT
Immunodétection du complexe PSA-ACT

(30) Priority: 31.07.1997 US 903749
(43) Date of publication of application: 03.02.1999
(73) Proprietor: Bayer Corporation, Pittsburgh, PA 15205-9741 (US)
(72) Inventor: Zhou, Zeqi, New City, New York 10956 (US); Allard, William Jeffrey, Poughquag, New York 12570 (US)
(74) Representative: Burkert, Frank

(56) References cited:
- EP-A- 0 838 680
- WO-A-95/18381
- WO-A-98/22509
- DE-A- 4 322 342
- CHICHIBU K ET AL: "SPECIFIC ENZYME IMMUNOASSAY FOR PROSTATE SPECIFIC ANTIGEN-ALPHA1-ANTICHYMOTRYPSIN COMPLEX (PSA-ACT) IN CLINICAL APPLICATIONS" JOURNAL OF UROLOGY, vol. 155, no. 5, May 1996, page 1 XP002061770
- WANG T I ET AL: "DEVELOPMENT OF MONOCLONAL ANTIBODIES SPECIFIC FOR THE PSA-ACT COMPLEX AND THEIR INCORPORATION INTO AN IMMUNOASSAY" CLINICAL CHEMISTRY, vol. 43, no. SUPPL. 06, 24 July 1997, page S225 XP002061771
- ZHOU Z ET AL: "TECHNICON IMMUNO 1 PSA ASSAY MEASURES BOTH FREE AND ALPHA-1-ANTICHYMOTRYPSIN-COMPLEXED PROSTATE-SPECIFIC ANTIGEN ON AN EQUIMOLAR BASIS" JOURNAL OF CLINICAL LABORATORY ANALYSIS, vol. 10, no. 3, 1996, pages 155-159, XP000674624
- CHICHIBU ET AL.: 'Fundamental evaluation of a detection kit for the complexed form of prostate specific antigen (PSA) with alpha-1-antichymotrypsin' JOURNAL OF MEDICINE AND PHARMACEUTICAL SCIENCE vol. 36, 1996, pages 477 - 483
- Hybritech: Instructions for the use of the ImmunoEnyMetric Assay, Tandem-E PSA

## Description

### BACKGROUND OF THE INVENTION

The present invention relates to the accurate and specific determination of the complex of prostate specific antigen (PSA) and alpha-1-antichymotrypsin (ACT) in a blood sample by a two-site immunometric assay.

Human prostate specific antigen (PSA) is a glycoprotein of approximately 33,000 daltons with high amino acid homology to the human kallikrein family (1,2) and has been shown to be a serine protease with trypsin and chymotrypsin-like activity (3,4,5). PSA is secreted by epithelial cells of the prostate gland and is one of the major proteins found in seminal fluid (6). Following the discovery that the concentration of PSA increases in the serum of patients with prostate cancer, numerous reports have established this protein as an important and clinically useful biomarker for the management of prostate cancer patients (7,8,9,10). Recent efforts have focused on the use of serum PSA testing for early detection of prostate cancer in asymptomatic men. In fact, the American Cancer Society and the American Urological Society have recently recommended that all men over the age of 50 be screened annually using serum PSA in conjunction with digital rectal examination (DRE) (11).

The clinical value of early detection of prostate cancer remains controversial for several reasons. First, it is unclear whether treatment of prostate cancer at early stages will improve survival in the affected population. Clinical trials designed to address this issue are currently underway. Second, a clinical trial recently measured the effectiveness of serum PSA measurements in conjunction with digital rectal examination (DRE) for early detection of prostate cancer in men over 50 years of age (12). Of the 1060 patients who had either an abnormal DRE or an elevated PSA test, only 22% had prostate cancer. These data demonstrate that of all prostate biopsies are performed on men who do not have cancer. Since 30-50% of men over the age of 50 have evidence of prostate cancer on autopsy, the number of unnecessary prostate biopsies triggered by elevated PSA assays could be very high. This has consequences both in increased medical costs and increased morbidity associated with the biopsy procedure.

Several laboratories have shown independently that PSA forms complexes with protease inhibitors such as α₁-antichymotrypsin (ACT), α₂-macroglobulin, and α₁-antitrypsin (13-19). PSA in complex with ACT or α₁-antitrypsin or in free, uncomplexed form is detectable in serum by immunoassay techniques. Indeed, the majority of immunoreactive PSA in serum is complexed with ACT, and a significant correlation has been established between the proportion of PSA bound to ACT and total serum PSA concentration (13). PSA bound to α₂-macroglobulin, however, is not measurable in serum due to steric hindrance of antibody binding to PSA following complexation with this protease. In early work, PSA-ACT levels and the proportion of PSA-ACT to total PSA were suggested to be of use in prostate cancer diagnosis (13,15,16,17), however, for a variety of reasons (some of which are discussed below) it has been difficult to draw conclusions on the clinical utility of serum measurement of PSA-ACT.

Lilja, Stenman, and coworkers published in 1991 that serum PSA exists in free form and in complexes with ACT and α₁-antitrypsin (13,18). In subsequent work, Stenman *et al*. demonstrated that measurement of PSA-ACT in association with measurement of free plus complexed PSA (termed total PSA, although PSA complexed with α₂-macroglobulin is not measured by conventional PSA assays) may improve discrimination between men with prostate cancer and those with benign prostate disease such as benign prostatic hypertrophy (BPH). However, the accurate measurement of PSA-ACT complexes has not been attainable due to technical problems in accurate measurement of the complex. Stenman *et al*. found that the correlation of PSA-ACT values with total PSA measurement was not good at the low end and the y intercept was elevated indicating over-recovery of complexed PSA (13 and U.S. Patent No. 5,501,983). Indeed, they found that for most patients tested, the concentration of PSA-ACT was higher than for total PSA (U.S. Patent No. 5,501,983). Subsequent correlation analysis for complexed and free PSA showed a slope of 1.12 indicating over-recovery of the PSA-ACT complex (16). Pettersson *et al*. addressed this over-recovery when they found elevated PSA-ACT values in female sera (20). While the addition of heparin reduced the incidence of false positive values in female serum, more recent attempts to measure PSA-ACT complexes in patients with prostate cancer and BPH continue to show significant over-recovery of complexes (21).

Because of the difficulties encountered in the measurement of PSA-ACT complexes, attention in the literature turned to the measurement of free, uncomplexed PSA in conjunction with measurement of total PSA. It is now clear that improved specificity is needed when total PSA values range from about 4-10 ng/mL. When serum total PSA is <4.0 ng/mL, the risk of prostate cancer is low; similarly, when total PSA is >10 ng/mL, the risk of prostate cancer is >50% and prostate biopsy is indicated. Within the diagnostic gray zone (generally between 2-20 ng/mL, more commonly between 4-10 ng/mL) the risk of cancer is high, but the rate of false positives is also high. The retrospective application of a ratio of free PSA/total PSA has shown that the specificity of total PSA in the 4-10 ng/mL gray zone could be improved from approximately 50-60% to 70-80% (22-26). This improved specificity could result in a 20-30% decrease in unnecessary biopsies. PCT WO 96-26441 and WO 97-12245 similarly describe the use of the free PSA/total PSA ratio to improve discrimination between BPH and cancer, respectively, in patients with total PSA levels between 2.5 and 20 ng/mL.

The measurement of free PSA has technical difficulties of its own, however. First, within the diagnostic gray zone, the proportion of free PSA is typically quite low, in the 5-30% range. A successful free PSA assay must, therefore, measure accurately in the range of 0.2-3.0 ng/mL. Also, the concentration of free PSA is not significantly different in patients with BPH and cancer, and the ratio of free PSA/total PSA decreases due to an increase in the proportion of PSA complexed to ACT. In addition, free PSA is not stable in serum and levels of free PSA have been known to decrease over time, presumably due to complexation with α₂-macroglobulin.

In the meantime, there has been further acknowledgment of the problems associated with die accurate measurement of PSA-ACT in blood, coupled with attempts to overcome such problems. In 1994, workers at Hybritech reported the development of a sandwich immunoassay for PSA-ACT employing anti-PSA and anti-ACT antibodies. They concluded that the measured PSA-ACT values failed to demonstrate improved clinical specificity in the diagnosis of prostate cancer (27). Later, this group jointly with workers at the Johns Hopkins Medical Institutions reported the finding that the anti-PSA/anti-ACT sandwich immunoassay method suffers from significant non-specific binding and over recovery of PSA-ACT. Unless resolved, they concluded that these problems rendered the measurement of PSA-ACT clinically meaningless (28). Subsequently, this joint group reported having overcome the non-specific binding problem through the development of a sandwich immunoassay for PSA-ACT based on a monoclonal antibody specific for PSA-ACT complex (29, 30). However, their clinical studies failed to show any improvement in specificity for prostate cancer by measuring PSA-ACT complex alone compared with measurement of total PSA or with a calculated ratio of PSA-ACT to total PSA (29). Other approaches to overcoming the problems associated with PSA-ACT measurement have been reported, including the use of blocking agents (31).

It remains unclear why the proportion of PSA complexed to ACT increases in patients with prostate cancer, but it may be related to the observation that antibodies to ACT do not stain prostatic epithelium from BPH patients and mRNA transcripts are not found in such tissue. In contrast, anti-ACT immunoreactivity and mRNA synthesis are detected in prostatic epithelium from patients with prostate cancer (32). These results suggest that in prostate tumors, PSA may complex *in situ* with ACT prior to release into serum. An alternative mechanism may involve the access of active PSA to the blood stream. Free PSA found in serum from healthy men is proteolytically cleaved and enzymatically inactive. Tumors, however, synthesize angiogenic factors which lead to increased vascularization of tumor tissues. It may be that in tumors, a larger proportion of enzymatically active PSA gains access to the blood stream. This active PSA would be expected to complex with protease inhibitors such as ACT leading to a higher proportion of PSA-ACT complex in serum from prostate cancer patients.

Accordingly, there is a need for an accurate method of determining complexed PSA and to assess the clinical significance of blood levels of complexed PSA relative to screening of male patients for prostate cancer.

EP 635,575 describes the preparation of monoclonal antibodies that bind to free PSA but not PSA-ACT.

PCT WO 95/18381 relates to a monoclonal/polyclonal immunometric assay method for the determination of PSA which is rendered capable of providing an equimolar response to free and complexed PSA by the addition of antibody that binds to free PSA but not complexed PSA.

U.S. Patent Application Serial No. 08/595,155 and Zhou Z, Ng PC, Very DL, Allard WJ, Yeung KK, J. Clin. Lab. Anal. (1996), 10:155-159, describe a method for preparing a monoclonal antibody that provides an equimolar response to free and complexed PSA in a monoclonal/polyclonal immunometric assay. The described monoclonal antibody has the unique property of binding to PSA to render PSA substantially incapable of binding with antibodies that bind free PSA but not complexed PSA.

Published Japanese Patent Document 62-46263 describes a sandwich immunoassay method for the determination of PSA in complex with protease inhibitor.

Published German Patent Application 4,322,342 describes a method for measuring both total PSA and PSA-ACT in a single assay for the purpose of providing values for calculation of the ratio of PSA-ACT to total PSA.

Chichibu *et al*, in the Journal of Medicine and Pharmaceutical Science (Japan, 1996) 36(3): 477-483, describe a sandwich immunoassay for PSA-ACT employing anti-PSA bound to a bead and enzyme-labeled anti-ACT. Data establishing the ability to accurately measure PSA-ACT in a blood sample is lacking.

### SUMMARY OF THE INVENTION

It has now been found that the accurate and specific determination of PSA-ACT in a blood sample can be obtained by a two-site immunometric assay in which the capture antibody is immobilized to microparticles (e.g., magnetically responsive microparticles). In general terms, the present method comprises the steps of (a) forming a reaction mixture containing immobilized and labeled immunocomplexes of PSA-ACT from the blood sample bound to a first antibody reagent specific for binding to PSA and to a second antibody reagent specific for binding to ACT, wherein one of said first and second antibody reagents is immobilized to microparticles having a mean diameter of between 0.01 µm and 100 µm ("capture antibody"), and the other of said antibody reagents is labeled ("detection antibody"), (b) separating the microparticles comprising said immobilized immunocomplexes from the remainder of said reaction mixture, (c) washing the separated microparticles, preferably at least twice, and (d) measuring the labeled antibody reagent in the washed microparticles. Preferably, the wash and separation steps are performed by an automated analyzer. Also, preferably, the first and second antibody reagents comprise monoclonal antibodies. The reaction mixture containing immobilized and labeled immunocomplexes of PSA-ACT is usually formed by mixing the blood sample with said first and second antibody reagents, wherein one of said first and second antibody reagents is immobilized, or capable of being immobilized, to said microparticles ("capture antibody"), and the other of said antibody reagents is labeled, or capable of being labeled ("detection antibody"), e.g., with an enzyme or chemiluminescent compound. In a particularly preferred method, the capture antibody is modified with a ligand and is immobilized by contact with a binding partner for the ligand fixed to said microparticles.

The present method provides specific and accurate detection of PSA-ACT complexes. As demonstrated in the examples which follow, the quantitation of PSA-ACT has been shown to be accurate because the sum of the measured values for free PSA plus PSA-ACT is equivalent to the measured value for total PSA. The difficulties reported in the literature in measuring PSA-ACT using manual formats, usually based on microtiter plate separation, are overcome by the use of a microparticle-based separation, preferably involving multiple washes, and preferably being performed on an automated analyzer. The present method has also been shown by spiked recovery experiments to accurately measure PSA-ACT both in a buffered solution and in serum. In addition, testing of serum samples from healthy men age 50 years or older, patients with BPH, and patients with prostate cancer has shown that the present method has clinical utility in the discrimination of men with and without prostate cancer. The upper limit of normal of the PSA-ACT assay was established to provide equivalent sensitivity to that of the prior art total PSA assays, Using this upper limit of normal (3.75 ng/mL), the specificity of the PSA-ACT assay for the detection of CaP in males whose total PSA levels are within the prior recognized diagnostic gray zone (e.g., between about 4-10 ng/mL) has been found to be comparable to that of recently developed methods based on the calculation of an fPSA/tPSA ratio.

The present method therefore provides a particularly useful aid in the detection of CaP in male human patients. Significantly, the present method aids in the decision whether to recommend that a male patient undergo costly and discomforting prostate biopsy. In these regards, the present method has the clear advantage of requiring the performance of only a single assay rather than dual assays as required in order to calculate an fPSA/tPSA ratio.

### BRIEF DESCRIPTION OF THE DRAWINGS

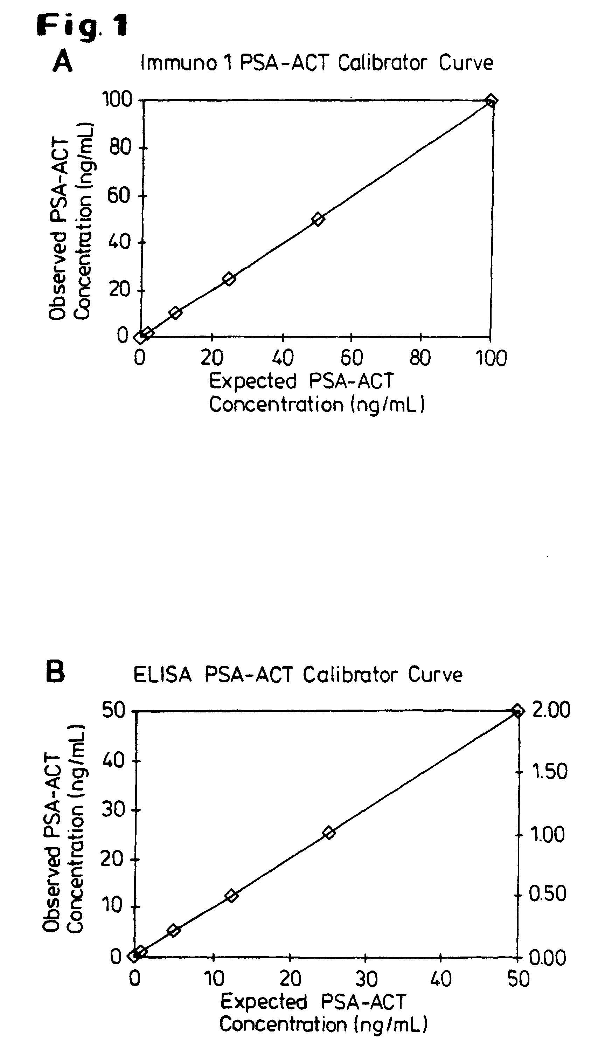

Figs. 1A and 1B are graphs showing standard curves for PSA-ACT using the PSA-ACT microparticle immunoassay and microplate-based ELISA PSA-ACT sandwich immunoassay, respectively.

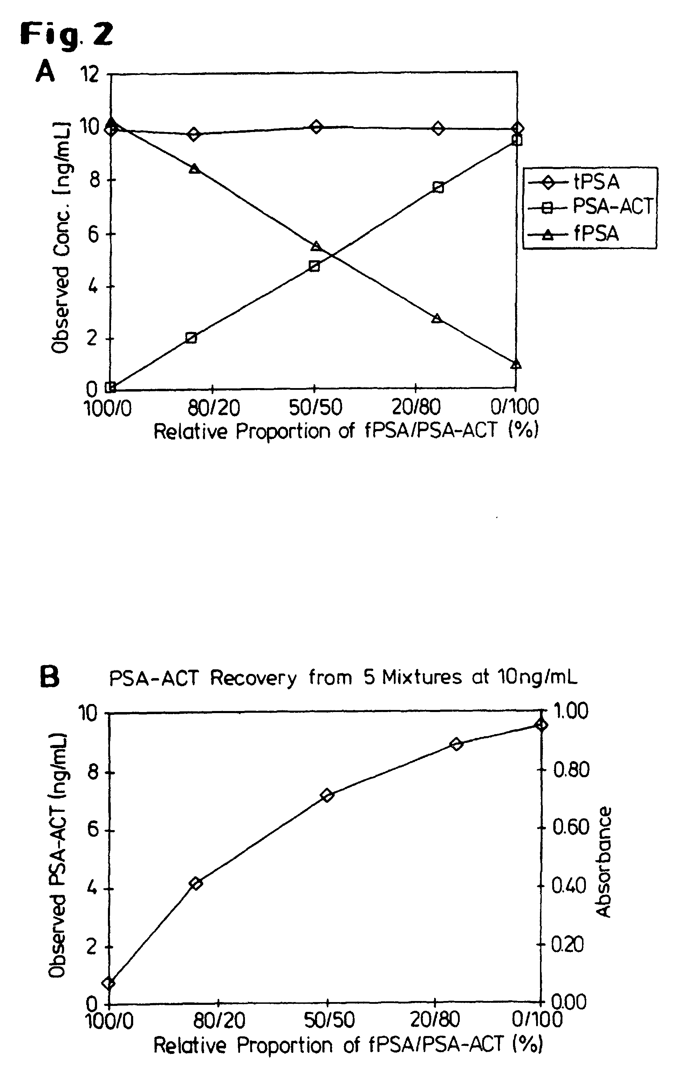

Figs. 2A and 2B are graphs showing the specificities of various PSA immunoassays. Samples contained approximately 10 ng/mL total PSA with varying proportions of free and PSA-ACT. In Fig. 2A it is shown that the PSA-ACT microparticle immunoassay measures PSA-ACT specifically and accurately. In addition, tPSA assay measures PSA and PSA-ACT with an equimolar response, and the fPSA assay measures free PSA specifically. The graph shown in Fig. 2B demonstrates the recovery of PSA-ACT in a microplate-based ELISA PSA-ACT immunoassay.

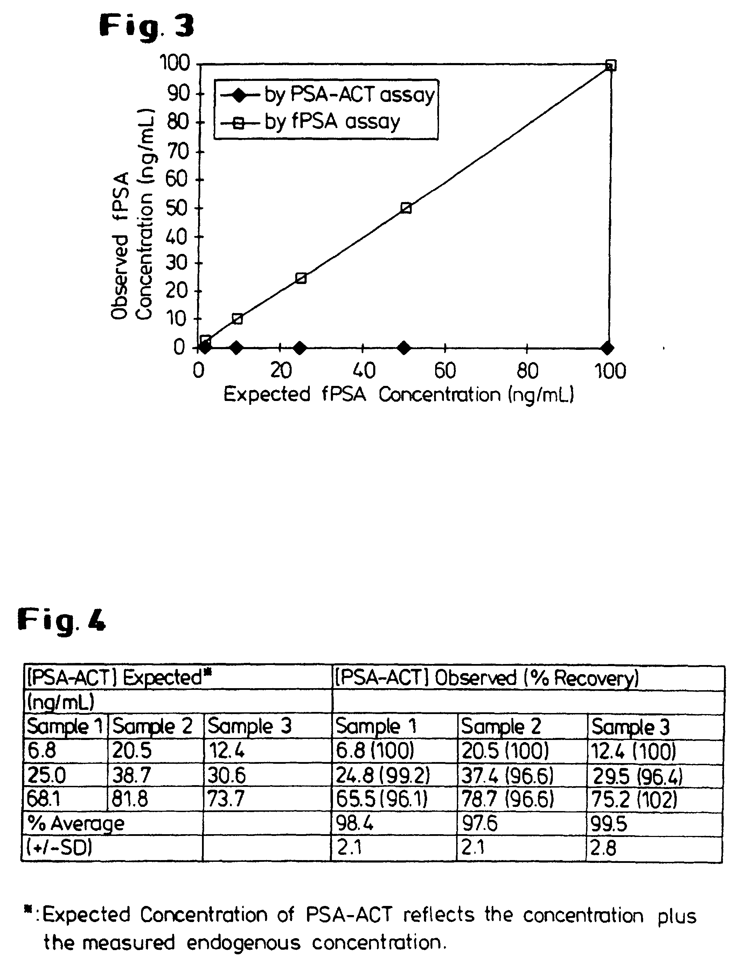

Fig. 3 is a graph showing the specificity of the PSA-ACT microparticle immunoassay described in the Examples. Samples containing fPSA at the concentrations of 0, 2, 10, 25, 50 and 100 ng/mL in the MES buffer containing 6% BSA were tested with a fPSA assay and the PSA-ACT assay. The data demonstrate that almost no free PSA was measured by the PSA-ACT microparticle-based immunoassay, but fPSA was fully quantitated by the fPSA assay.
Fig. 4 is a table of data showing that the PSA-ACT microparticle-based assay accurately measures PSA-ACT spiked into serum.

### DESCRIPTION OF THE PREFERRED EMBODIMENTS

As used herein, the following terms shall have the indicated meanings:
PSA shall mean prostate specific antigen.
tPSA or total PSA shall mean the total amount of immunologically determinable PSA in a blood sample, that is, PSA in complexed or free forms that are capable of responding to measurement by conventional immunoassays. Based on current knowledge, it is understood that blood PSA that is complexed with certain protease inhibitors (including ACT, α₁-antitrypsin, and inter-α trypsin inhibitor) is immunologically determinable, whereas PSA is not determinable when complexed with such other protease inhibitors as α₂-macroglobulin.
fPSA or free PSA shall mean PSA in its free, uncomplexed form.
PSA-ACT shall mean PSA complexed with ACT.

Antibody shall mean whole immunoglobulin, e.g., IgG or IgM, or an immunoglobulin fragment comprising an antibody binding site, e.g., Fab, Fab', and F(ab')₂ fragments, or aggregates thereof.

The present invention provides means for determining PSA-ACT in a blood sample by a two-site microparticle-based immunometric assay. It will be evident to one of ordinary skill in the art that a variety of two-site immunometric formats and microparticles having a mean diameter of between 0.01 µm and 100 µm can be used to measure PSA-ACT. In general, two antibodies are employed in a two-site immunometric assay method (sometimes referred to as a "sandwich" method). One of the antibodies is labeled or labelable (sometimes referred to as the "detection antibody") and the other is immobilized or immobilizable (sometimes referred to as the "capture antibody"). As is known in the art, the capture and detection antibodies can be contacted simultaneously or sequentially with the test sample. Sequential methods can be accomplished by incubating the capture antibody with the sample, and adding the labeled antibody a predetermined time thereafter (sometimes referred to as the "forward' method); or the detection antibody can be incubated with the sample first and then the labeled antibody added (sometimes referred to as the "reverse" method). After the necessary incubation(s) have occurred, to complete the assay, the capture antibody is separated from the liquid test mixture, and the label is measured in at least a portion of at least one of the separated capture antibody phase or the remainder of the liquid test mixture, normally the former since it comprises PSA bound by ("sandwiched" between) the capture and detection antibodies.

In typical two-site immunometric assays for PSA, one or both of the capture and detection antibodies are monoclonal antibodies. The label used in the detection antibody can be selected from any of those known conventionally in the art. Commonly, the label is an enzyme or a chemiluminescent moiety, but can also be a radioactive isotope, a fluorophor, a detectable ligand (e.g., detectable by a secondary binding by a labeled binding partner for the ligand), and the like. The important property of the capture antibody is that it provides a means for being separated from the remainder of the test mixture. Accordingly, as is understood in the art, the capture antibody can be introduced to the assay in an already immobilized or insoluble form, or can be in a immobilizable form, that is, a form which enables immobilization to be accomplished subsequent to introduction of the capture antibody to the assay. An example of an immobilizable capture antibody is antibody which has been chemically modified with a ligand moiety, e.g., a hapten, biotin, or the like, and which can thus be subsequently immobilized by contact with an immobilized form of a binding partner for the ligand, e.g., an antibody, avidin, or the like.

In accordance with the present invention, it has been found that the inaccuracies in the prior art methods for determining PSA-ACT are overcome by the use of microparticles having a mean diameter of between 0.01 µm and 100 µm as the means for immobilization of the capture antibody (either by direct immobilization of the capture antibody to microparticles or by indirect immobilization through the use of ligand-modified antibody with addition of microparticle-borne binding partner). As is well-known in the art, immobilization can be accomplished by a variety of means, such as covalent or noncovalent attachment. Moreover, it is preferred to subject the microparticles carrying the immobilized immunocomplexes of PSA-ACT bound to the capture and detection antibodies to multiple washings, e.g., at least 2 or more, in order to assure removal of background interferences. After separating the microparticles from the remainder of the reaction mixture, they are mixed with a first wash liquid, e.g., buffer, separated from the first wash liquid, mixed with a second wash liquid, e.g., further buffer, separated again, and so forth for the desired number of washes. The wash and separation steps are most preferably performed by an automated analyzer in order to obtain consistency of washing.

The above-described immunoassay methods and formats are intended to be exemplary and are not limiting since, in general, it will be understood that any two-site microparticle-based immunoassay method or format can be used in the present invention.

A wide variety of microparticles can be used in the present invention. In general, the microparticles will have a mean diameter of between 0.01 µm and 100 µm in order to promote highly efficient washing and thereby removal of potentially interfering amounts of nonspecifically bound ACT from the blood sample. The microparticles will have a mean diameter of between about 0.01 microns and about 100 microns, more usually between about 1 micron and about 50 microns. The microparticles can be composed of a wide variety of natural and/or synthetic materials and can be prepared in a wide variety of ways. Although they may be of any particular shape, essentially spherical microparticles are typically known and used in immunoassay applications. Commonly, the microparticles are in the form of latexes, colloids, hydratable gel particles, and the like. For example, a wide variety of latex microparticles are available commercially, such as from Bangs Laboratories, Carmel, Indiana, USA or Dow Chemical Company, Midland, Michigan, USA. Such latex microparticles are typically composed of such polymers as polystyrene, butadiene styrenes, ployethylmethacrylate, polyvinyl acetate, polyvinylpyridine, polybutyleneterephthalate, and functional derivatives thereof, e.g., bearing such groups as aldehyde, carboxy, amino, hydroxyl, or hydrazide, which serve as sites of chemical coupling of a desired antibody reagent.

Particular advantage can be taken by the use of microparticles which are magnetically responsive, e.g., ferromagnetic or paramagnetic microparticles. Magnetically responsive microparticles will comprise a metal (such as iron, nickel or cobalt), a metal alloy (such as metallic alloys of aluminum, nickel or cobalt), and/or a metal oxide (such as Fe₃O₄ or Fe₂O₃). In preferred form, such magnetically responsive microparticles also comprise a nonmagnetic polymeric matrix or coating. Examples of useful magnetically responsive microparticles are described in U.S. Patent Nos. 4,177,253; 4,661,408; 5,206,159; 5,320,944; 5,541,072; and 5,567,326. The polymer particles containing iron oxide granules described in U.S. Pat. No. 5,206,159 are particularly preferred.

The anti-PSA and anti-ACT antibodies employed in the present method can be obtained by any conventional means, including both polyclonal antibodies obtained by harvesting antiserum from an appropriately immunized host animal and monoclonal antibodies, which are preferred, obtained by somatic cell hybridization. Somatic cell hybridization is now a well-known methodology and can be applied to the present invention in all of its variations as appropriate and desired. In general, one prepares a population of hybridomas by fusion of myeloma cells with lymphocyte cells taken from an animal that has been immunized against the analyte. Immunization of the host animal, as used herein, implies that the animal's immune system has been challenged to produce antibodies that will bind to one or more epitopes on the analyte of interest. It will be evident to the skilled worker in the art that such result can be obtained in any number of ways, including, without limitation, administration of the native analyte, synthetic peptide immunogen, tranfectant cells which express epitopes of the analyte on their surface, or the like, to the bloodstream of the host animal. Similarly, production and harvesting of monoclonal antibodies from cloned hybridoma cell lines are within the ordinary skill in the art, and in general any known method can be used in practicing the present invention.

The reagents and other assay components necessary for practice of the above-described particularly preferred method for determining PSA-ACT are conveniently provided in the form of a test kit, that is, a packaged collection or combination as appropriate for the needs of the user and any analytical instrumentation involved. Minimally, the test kit will comprise the aforesaid first and second antibodies.

PSA-ACT blood values in male patients provide substantial clinical significance in comparison to the prior art tPSA values and fPSA/tPSA ratio values. Specifically, a study was conducted using serum samples from 226 patients including 53 patients with CaP, 75 patients with BPH, and 88 healthy male controls over the age of 50 years. In this study, measurement of PSA-ACT was found to give equivalent sensitivity for detection of CaP as compared with measurement of tPSA (85% v. 88%, respectively). For all patients tested, the specificity in the normal and BPH populations was also comparable for compared to the fPSA/tPSA ratio. The finding that the sensitivity and specificity of the tPSA assay used in conjunction with a fPSA/tPSA ratio is equivalent to that of the PSA-ACT alone also held true when the patient population was stratified into the diagnostic gray zone. The precise range of the diagnostic gray zone has not been defined, but at all ranges compared in this study, the sensitivity and specificity of the PSA-ACT assay was comparable to that obtained using both total and free PSA assays. These data demonstrate that a single test, PSA-ACT, can detect prostate cancer as efficiently as total PSA, and, in addition, has the improved specificity that has been shown to be obtainable using two assays, fPSA and tPSA.

In addition to the above-discussed use in the detection of prostate cancer, the measurement of PSA-ACT will be useful in the monitoring of the course of disease in patients who have been diagnosed with prostate cancer, particularly after having received first line therapy for prostate cancer. Longitudinal monitoring of such patients by measurement of tPSA has been proven to be useful in the early detection of recurrent prostate cancer. PSA-ACT is understood to be the cancer-specific form of PSA and is the form that would be expected to increase in serum as cryptic cancer cells establish distant metastic sites and grow. Accordingly, changes in PSA-ACT blood levels over time will correlate with changes in disease status, and particularly, increasing PSA-ACT blood levels after therapy will indicate recurrence of disease.

The present invention will now be illustrated, but is not intended to be limited by, the following examples.

### EXAMPLES

### MATERIALS AND METHODS

### Antibodies and Antigens

Antibodies used in this study include the following: MM1, a monoclonal antibody specific for PSA which recognizes both free and complexed PSA (substantially similar antibodies are those produced by hybridoma cell lines 346.7.4 and 346.7.26 deposited with the American Type Culture Collection, Rockville, Maryland, USA, on April 10, 1997, and assigned deposit numbers HB-12338 and 12337, respectively); and MP2, an affinity purified polyclonal antibody to PSA derived from goats. A free PSA-specific monoclonal antibody, PSA19, and two ACT-specific monoclonal antibodies, ACT 53 (Lot # 6306) and ACT 68 (Lot # 6268), were purchased from CanAg Diagnostics AB (Gothenburg, Sweden). These antibodies were purified using Protein-A affinity chromatography, and stored in TRIS-HCl buffer, pH 7.8 containing 0.9% sodium chloride.

Antigens used in these studies include free PSA (Lot #, 751464) and PSA-ACT complex (Lot #, 145564) purchased from Scripps Laboratories (San Diego, California, USA). Free PSA was purified from human seminal fluid with 98% purity as determined by SDS-PAGE and enzyme immunoassays and stored in 10 mM TRIS buffer, pH 8.0, containing 0.1% sodium azide. ACT was purified from human plasma to approximately 96% purity. PSA-ACT complex was stored in 10 mM sodium acetate buffer, pH 5.6, containing 150 mM sodium chloride and 0.1% sodium azide. Free PSA and PSA-ACT antigens were used for the preparation of the tPSA, fPSA, and PSA-ACT calibrators.

### Buffers and Solutions

A buffer for use with antibody conjugates was prepared using 100 mM TRIS-HCl, pH 7.4, with 5% heat-inactivated normal goat serum (Biocell Laboratories, Carson, California, USA). 25 mM TRIS-HCl buffer, pH 7.4, containing 111 mM sodium chloride and 6% BSA was used to prepare calibrators for the Bayer Immuno 1™ total PSA and free PSA assays (Bayer Corporation, Business Group Diagnostics, Tarrytown, New York, USA). MES buffer (50 mM), pH 5.8, containing 111 mM sodium chloride and 6% BSA was used to prepare calibrators for the PSA-ACT assay.

### Calibrators and Controls

PSA-ACT calibrators were prepared at six concentrations: 0, 2, 10, 25, 50, and 100 ng/mL, using the 50 mM MES buffer. These calibrators were used in both the Bayer Immuno 1 PSA-ACT assay and the manual PSA-ACT ELISA assay (both as described below). Purified free PSA from human seminal fluid was suspended at the same six concentrations in the TRIS buffer described above for use in the total and free PSA assays.

Two sets of PSA-ACT controls were prepared. One set contained two concentrations of PSA at 4 and 25 ng/mL using 90% PSA-ACT and 10% free PSA using the TRIS-HCl buffer as a diluent. A second set of controls was prepared using three concentrations of PSA-ACT of 3.5 , 15 and 75 ng/mL using the 50 mM MES buffer. These two control preparations were used in the Bayer Immuno 1 PSA-ACT assay and the manual PSA-ACT ELISA. A commercial tri-level control product was obtained from BioRad Laboratories, California, USA. A control product using human serum spiked with free PSA to a final concentration of 4 ng/ml was prepared at Bayer Corporation. The latter two materials were used as controls for the Bayer Immuno 1 tPSA and fPSA assays.

### Serum Samples

A total of 216 serum samples from 53 patients with prostate cancer and from 163 BPH patients and normal males age 50 years or older were obtained from BioClinical Partners (Franklin, Massachusetts, USA). The diagnoses of prostate cancer and BPH were confirmed by histology, and men were considered normal with regard to prostate disease on the basis of a normal digital rectal examination.

### The Bayer Immuno 1™ PSA Assay

The Bayer Immuno 1 PSA assay is a commercially available sandwich assay which uses a monoclonal antibody for capture and polyclonal antibodies for detection of PSA. The monoclonal anti-PSA antibody is conjugated to fluorescein (R1, 1.5 mg/mL) and the affinity-purified polyclonal antiserum is conjugated to alkaline phosphatase (R2, 6.5 mg/mL). Solutions of the two antibodies, at 65 ml/test, are incubated with specimen to form an immunocomplex (R1-PSA-R2) which is then captured by magnetic particles (10 ml/test) coated with anti-fluorescein monoclonal antibodies. The particle bound immunocomplexes are washed and then 300 ml/test of 23 mM p-nitrophenyl phosphate are added and the rate of its conversion by bound alkaline phosphatase to p-nitrophenolate is measured. Calibration of the Bayer Immuno 1 analyzer is performed using the Bayer Immuno 1 SET point® PSA calibrators, prepared from free PSA at concentrations of 0, 2, 10, 25, 50 and 100 ng/mL. A cubic-through-zero fitting algorithm is used to generate a standard calibration curve. Operation of the Bayer Immuno 1 analyzer is performed according to the manufacturer's instructions (Bayer Corporation, Tarrytown, New York, USA).

### Microparticle-Based Assay for PSA-ACT

The Bayer Immuno 1 PSA-ACT assay format is the same as that of the Bayer Immuno 1 tPSA Assay except for the following changes: (1) a monoclonal antibody specific for ACT, ACT 53, is used for detection at 2 µg/mL; (2) PSA-ACT is used as the calibrator and control antigen with the 50 mM MES buffer, pH 5.8, as a base; and (3) a two-wash protocol is used such that antigen is first incubated with capture antibody, the resulting complex is washed to remove unbound antigen and other serum components, and then the detection antibody is added. This is in contrast to the format used in the Bayer Immuno 1 tPSA assay where antigen is incubated simultaneously with the capture and detection antibodies.

### Automated Assay for free PSA

The protocol used for the Bayer Immuno I fPSA assay is the same as that described above for the Bayer Immuno 1 tPSA assay except the following changes: (1) monoclonal antibody, PSA19 specific for fPSA, is conjugated to fluorescein for capture; (2) the concentrations of R1 and R2 used are 2.5 µg /mL and 6.15 µg/mL, respectively; and (3) the sample volume is increased from 20 µl to 35 µl per test.

### Manual Microplate ELISA Method for PSA-ACT

Immulon 1® microtiter plates (Dynatech Laboratories, Chantilly, Virginia, USA) were coated with the MM1 capture antibody using 100 µl/well at 1.5 µg/mL in 100 mM sodium carbonate buffer, pH 9.6. The plates were incubated at room temp for 1.5 hr. Unreacted sites were quenched with 300 µl/well of TRIS, pH 7.4 containing 6% BSA, and incubated overnight at 4°C. A 100 µl volume of either PSA-ACT calibrators, controls, or patient samples was added and incubated at room temperature for 1.5 hours. After 5 washes with TRIS buffered saline using an Ultrawash II Microplate Washer (Dynatech Laboratories, Chantilly, Virginia USA), ALP-ACT #53 detection antibody (100 µl/well) in GS5 buffer was added and incubated for 1.5 hours at RT. After 5 more washes 100 µl of p-nitrophenyl phosphate substrate solution (1 mg/mL) in diethanolamine (Pierce Chemicals, Rockford, Illinois, USA) was added and incubated at room temperature. The immunoreaction was stopped with the addition of 100 µl/well 1N sodium hydroxide. Absorbance of each well was read at 405-490 nm on Thermo-Max Microplate Reader (Molecular Devices, Menlo Park, California, USA). To assure accurate quantitation of PSA-ACT, a calibration curve was run in each microplate. The absorbance was converted to analyte concentration using a cubic fit through zero algorithm. All samples were run in duplicate.

### RESULTS AND DISCUSSION

### Selection of Antibodies for the measurement of PSA-ACT

Two monoclonal antibodies, ACT 53 and ACT 68, specific for ACT, were selected for analysis as detection antibodies. ACT 53 was used at 2 µg/mL and ACT 68 was used at 4 µg/mL in combination with the capture antibody, MM1, to form a sandwich assay for the measurement of PSA-ACT. Reaction rates of the PSA-ACT calibrators, and the analyte concentrations of 15 prostate cancer patient serum samples were compared using the two anti-ACT antibodies. Results showed that ACT 53 gave reaction rates which were ≥52% higher than that for ACT 68. Furthermore, background signal using ACT 68 was significantly higher than that seen with antibody ACT 53. Because antibody ACT 53•demonstrated a higher signal to noise ratio, it was selected for further analysis.

### Standard Curves of Both Bayer Immuno 1 PSA-ACT and ELISA PSA-ACT Assays

A standard curve was derived using the six-level PSA-ACT calibrators run on the automated Bayer Immuno 1 PSA-ACT Assay and results are shown in Figure 1A. A standard curve was also derived using the six-level PSA-ACT calibrators run in the manual microplate PSA-ACT Assay and results are shown in Figure 1B. In both cases, the signal was proportional to the concentration of PSA-ACT.

### Recovery of PSA-ACT from Mixtures Containing Free PSA and PSA-ACT

Five mixtures various proportions of free and ACT-complexed PSA at free PSA:PSA-ACT ratios of 100:0, 80:20, 50:50, 20:80 and 0:100 were diluted to a total PSA concentration of approximately 10 ng/mL using the same TRIS buffer used to prepare the PSA-ACT calibrators. These samples were run in each of three assays on the Bayer Immuno I analyzer: total PSA, free PSA, and PSA-ACT. To measure total and free PSA, the Bayer Immuno I system was calibrated with calibrators prepared using free PSA. For the measurement of PSA-ACT, the Bayer Immuno 1 system was calibrated using calibrators prepared with PSA-ACT. The automated Bayer Immuno 1 PSA-ACT Assay used the ACT 53 detection antibody as described above. Results in Figure 2A show that the Bayer Immuno 1 PSA-ACT assay measures PSA-ACT accurately, does not detect free PSA, and has no apparent problem with over-recovery of PSA-ACT as has been reported for previously described assays measuring PSA-ACT.

In addition, the specificity of the solid phase microplate-based ELISA PSA-ACT assay was determined to compare with that of the Bayer Immuno 1 microparticle-based PSA-ACT assay. Samples containing approximately 10 µg/mL total PSA with various proportions of free PSA and PSA-ACT as described above, were tested in the solid phase microplate-based ELISA PSA-ACT assay. Results shown in Fig. 2B demonstrate that recovery of PSA-ACT was not linear with PSA-ACT concentration, and that PSA-ACT measurement was inaccurate in this assay, particularly at low concentrations of PSA-ACT. This is in marked contrast to the recovery of PSA-ACT in the microparticle-based assay. As an example, the 80:20 sample should contain 20% PSA-ACT and should read 2 ng/mL in an immunoassay. Recovery of this sample in the microparticle-based assay was approximately 2 ng/mL, as expected. In contrast, the microplate-based ELISA measured the concentration of PSA-ACT in this sample as 4 ng/mL, which is 100% higher than expected. Repeated manipulations of the microplate-based ELISA assay did not correct this over-recovery problem.

### Specificity of the Bayer Immuno 1 PSA-ACT Assay

Both free PSA and PSA-ACT at PSA concentrations of 0, 2, 10, 25, 50 and 100 ng/mL were tested as unknown samples using the automated Bayer Immuno 1 PSA-ACT Assay. Results in Figure 3 show that no free PSA was detected by the Bayer Immuno 1 PSA-ACT assay. However, the recovery of free PSA was linear with expected values as determined by the Bayer Immuno 1 fPSA assay. These results demonstrate that the Bayer Immuno 1 PSA-ACT Assay is specific for PSA-ACT with no detectable reactivity with free PSA.

### PSA-ACT Spiked Recovery from Patient Serum Samples.

PSA-ACT at three concentrations of 0, 18 and 61 ng/mL was spiked into three prostate cancer patient serum samples. The PSA-ACT concentrations in these three patient sera were measured using the Bayer Immuno 1 PSA-ACT assay, and compared to values obtained with the Bayer Immuno 1 total PSA assay which is equimolar and accurately measures both free and complexed PSA. Data shown in Fig. 4 indicate an average of 98% recovery of the spiked PSA-ACT for all 3 samples in the PSA-ACT Assay. Because free PSA is measurable in the total PSA Assay, but not in the PSA-ACT assay, the 2% difference in quantitation by the Bayer Immuno 1 total PSA Assay versus the Bayer Immuno 1 PSA-ACT Assay may be due to dissociation of free PSA from the PSA-ACT complex.

### Accuracy of PSA-ACT Measurement

The mean values for (free PSA + PSA-ACT)/total PSA using the Bayer Immuno 1 PSA-ACT assay were 96% in prostate cancer patient sera, 102.7% from BPH patient sera and 103% from normal age-matched serum samples. This indicates that no over-recovery of PSA-ACT in sera was obtained using the Bayer Immuno 1 PSA-ACT assay. In contrast, recoveries using the microplate-based ELISA PSA-ACT assay were approximately 171%, 135% and 164% for the same patient sera. These data confirm results published by other laboratories that microtiter plate ELISA measurement of PSA-ACT yielded higher than expected results (13, 14,16).

### BIBLIOGRAPHY

1. Wang MC, Valenzuela LA, Murphy GP, Chu TM. Purification of a human prostate specific antigen. Invest Urol 1979; 17:159-63.
2. Watt WK, Lee PJ, Timkulu TM, Chan WP, Loor R. Human prostate-specific antigen: structure and functional similarity with serine proteases. Proc Natl Acad Sci USA 1986; 83:3166-70.
3. Akiyama K, Nakamura T, Iwanaga S, Hara M. The chymotrypsin-like activity of human prostate-specific antigen, r-seminoprotein. FEBs Letters 1987; 225:168-72.
4. Christensson A, Laurel CB, Lilja H. Enzymatic activity of prostatic-specific antigen and its reactions with extracellular serine proteinase inhibitors. Eur J Biochem 1990; 194:755-763.
5. Lilja H. A kallikrein-like serine protease in prostatic fluid cleaves the predominant seminal vesicle protein. J Clin Invest 1985; 76:1899-903.
6. Lilja H, Abrahamsson PA. Three predominant proteins secreted by the human prostate gland. Prostate 1980; 12:29-38.
7. Papsidero LD, Wang MC, Valenzuela 1A, Murphy GP, Chu TM. A prostate antigen in sera of prostate cancer patients. Cancer Res 1980; 40:2428-2432.
8. Lange PH. Prostate specific antigen in diagnosis and management of prostate cancer. Supplement to Urology 1990; XXXVI:25-29.
9. Oesterling JE. Prostate specific antigen: a critical assessment of the most useful tumor marker for adenocarcinoma of the prostate. J Urol 1991; 145:907-923.
10. Armbruster DA. Prostate-specific antigen: biochemistry, analytical methods and clinical application. Clin Chem 1993; 39:181-195.
11. American Cancer Society: Cancer facts & figures-1995. American Cancer Society, Inc., Atlanta, GA. Page 11.
12. Catalona WJ, Richie JP, Ahmann FR, Hudson MA, Scardino PT, Flanigan RC, deKernion JB, Ratliff TL, Kavoussi LR, Dalkin BL, Waters, WB, MacFarlane MT and Southwick PC. Comparison of digital rectal examination and serum prostate specific antigen in the early detection of prostate cancer: results of a multicenter clinical trial of 6,630 men. J Urol 1994; 151:1283-1290.
13. Stenman UH, Leinonen J, Alfthan H, Rannikko S, Tuhkanen K, Alfthan O. A complex between prostate-specific antigen and α121-antichymotrypsin is the major form of prostate-specific antigen in serum of patients with prostatic cancer: assay of the complex improves clinical sensitivity for cancer. Cancer Res 1991; 51:222-226.
14. Zhou AM, Tewari PC, Bluestein BL, Caldwell, GW, Larsen FL. Multiple forms of prostate-specific antigen in serum: differences in immunorecognition by monoclonal and polyclonal assays. Clin Chem 1993; 39:2483-91.
15. Leinonen J, Lovgren T, Vornanen T, Stenman UH. Double-label time-resolved immunofluorometric of prostate-specific antigen and its complex with α₁-antichymotrypsin. Clin Chem 1993: 39:2098-2103.
16. Christensson A, Bjork T, Nilsson O, Nilsson O, Dahlen U, Matikainen MT, Cockett AT, Abrahamssion PA. Serum prostate specific antigen complexed with α₁-antichymotrypsin as an indicator of prostate cancer. J Urol 1993; 150:100-105.
17. Lilja H, Significance of different molecular forms of serum PSA. The free, noncomplexed forms of PSA versus that complexed to α₁-antichymotrypsin. Urol Clin North Am 1993; 20(4):681-686.
18. Lilja H, Christensson A. Dahlen U, Matikainen M-T, Nilsson O, Pettersson K and Lovgren T: Prostate-specific antigen in human serum occurs predominantly in complex with alpha-antichymotrypsin. Clin Chem 1991; 37:1618-1625.
19. Bjork T, Hulkko S, Bjartell A, Santagnese AD, Abrahamsson PA, Lilja H. Alpha₁-antichymotrypsin production in PSA-producing cells is common in prostate cancer but rare in benign prostatic hyperplasia. Urology 1994; 43:427-434.
20. Pettersson K, Piironen T, Seppala M, Liukkonen L, Christensson A, Matikainen M-T, Suonpaa M, Lovgren T, Lilja H. Free and complexed prostate-specific antigen (PSA): in *vitro* stability, epitope map, and development of immunofluorometric assays for specific and sensitive detection of free PSA and PSA-α₁-antichymotrypsin complex. Clin Chem 1995; 41:1480-1488.
21. Wu IT, Wilson L, Zhang P, Meikle AW, Stephenson R. Correlation of serum concentrations of PSA-ACT complex with total PSA in random and serial specimens from patients with BPH and prostate cancer. J Clin Lab Anal 1995 9:15-24.
22. Mitrunen K, Pettersson K, Piironen T, Bjork T, Lilja H, Lovgren T. (1995) Dual label one-step immunoassay for simultaneous measurement of free and total prostate specific antigen concentrations and ratios in serum. Clin Chem 1995 41(8):1115-1120.
23. Catalona WJ, Smith DS, Wolfert RL, Wang TJ, Rittenhouse HG, Ratliff TL, Nadler RB. Evaluation of percentage of free serum prostate-specific antigen to improve specificity of prostate cancer screening. JAMA 1995 274(15): 1214-1220.
24. Prestigiacomo AF, Stamey TA. Clinical usefulness of free and complexed PSA. Scan J Clin Lab Invest 1995 55Supple221:32-34.
25. Wang TJ, Hill TM, Sokoloff RL, Frankenne F. Rittenhouse HG, Wolfert RL. Dual monoclonal antibody immunoassay for free prostate-specific antigen. Prostate 1996 28:10-16.
26. Jung K, Stephan C, Lein M, Henkne W, Schnorr D, Brux B, Schurenkamper P, Loening SA. Analytical performance and clinical validity of two free prostate-specific antigen assays compared. Clin Chem 1996 42(7):1026-1033.
27. Wang TJ, Hill T, Sokoloff R, Frankenne F, Wolfert R, and Ritterhouse H. Monoclonal antibody sandwich immunoassay to quantitate free PSA in benign hyperplasia and prostate cancer. Poster presentation at 1994 ISOBM meeting.
28. Chan D, Kelley CA, Partin AW, Linton J, Wang TJ, Sokoloff RL, Rittenhouse HG, and Wolfert RL. Clin Chem (1996) 42(6):S255.
29. Wang TJ, Linton J, Payne J, Rittenhouse HG, Wolfert RL, Chan DW, Kelley CA and Partin AW. Clinical utility of a complexed PSA immunoassay with a specific monoclonal antibody to PSA-ACT. J Urology (1997) 157(4) Suppl:147.
30. Wang TJ, Linton HJ, Payne J, Liu R-S, Kuus-Reichel K, Rittenhouse HG, Kelley C, Cox J, Chan DW, and Wolfert RL. Development of monoclonal antibodies specific for the PSA-ACT complex and their incorporation into an immunoassay. Clin Chem (1997) 43(6):S225.
31. Zhang P and Wu, JT. Development of an immunoassay for the PSA-ACT complex in serum without interference of non-specific adsorption. Clin Chem (1997) 43(6):S236.
32. Bjork T, Bjartell A, Abrahamsson P-A, Hulkko S, Di Sant'agnese A, Lilja H. Alpha₁-antichymotrypsin production in PSA-producing cells is common in prostate cancer but rate in benign prostatic hyperplasia. Urol 1994 43(4):427-434.

## Claims

1. A process for performing a PSA-ACT assay using a blood sample, comprising:
(a) forming a mixture including a first antibody, a second antibody, PSA-ACT, and microparticles with a mean diameter of between 0.01 µm (microns) and 100 µm (microns);
(b) wherein the first antibody is capable of being immunologically bound with PSA of PSA-ACT;
(c) wherein the second antibody is capable of being immunologically bound with ACT of PSA-ACT;
(d) wherein each of the first antibody and the second antibody is capable of being immobilized to the microparticles or labelled with detectable compounds;
(e) forming complexes comprising the first antibody, the second antibody, and PSA-ACT, wherein (I) the first antibody is immunologically bound with PSA of PSA-ACT, (II) the second antibody is immunologically bound with ACT of PSA-ACT, and (III) the complexes are immobilized to the microparticles and labelled with the detectable compounds;
(f) separating the complexes within a portion of the mixture from the remainder of the mixture;
(g) generating signals relating to the detectable compounds;
(h) detecting the signals; and
(i) determining, based upon a proportional relationship between the detected signals and the concentration of PSA-ACT, the amount of PSA-ACT.

2. The process as defined by Claim 1, wherein:
(a) the microparticles have a mean diameter of between 1 µm (microns) and 50 µm (microns).

3. The process as defined by Claim 1, wherein:
(a) separating the complexes uses a wash liquid and is performed by an automated analyzer.

4. The process as defined by Claim 1, wherein:
(a) each of the first antibody and the second antibody is a monoclonal antibody.

5. The process as defined by Claim 1, wherein:
(a) the microparticles are in the form of latexes, colloids, or hydratable gels.

6. The process as defined by Claim 5, wherein:
(a) the microparticles are polymer latexes.

7. The process as defined by Claim 1, wherein:
(a) the microparticles are magnetically responsive.

8. The process as defined by Claim 7, wherein:
(a) the magnetically responsive microparticles are nonmagnetic polymer matrices containing iron oxide.

9. The process as defined by Claim 1, wherein:
(a) the detectable compounds are enzymatic, chemiluminescent, fluorescent, or radioactive.

10. The process as defined by Claim 1, wherein:
(a) each of the first antibody or the second antibody is modified with an immobilization-ligand;
(b) the microparticles contain an immobilization-ligand-binding-partner fixed thereon; and
(c) the complexes are immobilized to the microparticles through immunological binding between the immobilization-ligand and the immobilization-ligand-binding-partner.

11. A test kit for performing a PSA-ACT assay using a blood sample, comprising:
(a) a first antibody, a second antibody, microparticles with a mean diameter of between 0.01 µm (microns) and 100 µm (microns) and detectable compounds;
(b) wherein the first antibody is capable of being immunologically bound with PSA of PSA-ACT;
(c) wherein the second antibody is capable of being immunologically bound with ACT of PSA-ACT; and
(d) wherein each of the first antibody and the second antibody is capable of being immobilized to the microparticles or labelled with detectable compounds.

12. The test kit as defined by Claim 11, wherein:
(a) the microparticles have a mean diameter of between 1 µm (microns) and 50 µm (microns).

## Patentansprüche

1. Verfahren zur Durchführung eines PSA-ACT-Assay mit einer Blutprobe, umfassend Stufen, in denen man:
(a) eine Mischung bildet, die einen ersten Antikörper, einen zweiten Antikörper PSA-ACT und Mikropartikel mit einem mittleren Durchmesser von 0,01 bis 100 µm einschließt,
(b) worin der erste Antikörper dazu befähigt ist, immunologisch mit PSA von PSA-ACT gebunden zu werden,
(c) worin der zweite Antikörper dazu befähigt ist, immunologisch mit ACT von PSA-ACT gebunden zu werden,
(d) worin jeder des ersten und zweiten Antikörpers dazu befähigt ist, an den Mikropartikeln immobilisiert oder mit nachweisbaren Verbindungen markiert zu werden,
(e) Komplexe bildet, umfassend den ersten Antikörper, den zweiten Antikörper und PSA-ACT, worin (I) der erste Antikörper immunologisch mit PSA von PSA-ACT gebunden wird, (II) der zweite Antikörper immunologisch mit ACT von PSA-ACT gebunden wird, und (III) die Komplexe an den Mikropartikeln immobilisiert und mit den nachweisbaren Verbindungen markiert werden,
(f) die Komplexe innerhalb eines Teils der Mischung aus dem verbleibenden Teil der Mischung abtrennt,
(g) Signale erzeugt, die mit den nachweisbaren Verbindungen zusammenhängen,
(h) die Signale nachweist, und man,
(i) bezogen auf eine proportionale Beziehung zwischen den nachgewiesenen Signalen und der Konzentration von PSA-ACT, die Menge von PSA-ACT bestimmt.

2. Verfahren gemäß Anspruch 1, worin:
(a) die Mikropartikel einen mittleren Durchmesser von 1 bis 50 µm aufweisen.

3. Verfahren gemäß Anspruch 1, worin:
(a) bei der Abtrennung der Komplexe eine Wasch-Flüssigkeit verwendet wird und das Ganze in einem automatisierten Analysegerät durchgeführt wird.

4. Verfahren gemäß Anspruch 1, worin:
(a) jeder des ersten und zweiten Antikörpers ein monoklonaler Antikörper ist.

5. Verfahren gemäß Anspruch 1, worin:
(a) die Mikropartikel in der Form von Latices, Kolloiden oder hydratisierbaren Gelen vorliegen.

6. Verfahren gemäß Anspruch 5, worin:
(a) die Mikropartikel Polymer-Latices sind.

7. Verfahren gemäß Anspruch 1, worin:
(a) die Mikropartikel magnetisch reagibel sind.

8. Verfahren gemäß Anspruch 7, worin:
(a) die magnetisch reagiblen Mikropartikel nicht-magnetische Polymer-Matrices sind, die Eisenoxid enthalten

9. Verfahren gemäß Anspruch 1, worin:
(a) die nachweisbaren Verbindungen enzymatisch, chemilumineszent, fluoreszent oder radioaktiv sind.

10. Verfahren gemäß Anspruch 1, worin:
(a) jeder der ersten oder zweiten Antikörper mit einem Immobilisierungsliganden modifiziert ist;
(b) die Mikropartikel einen daran fixierten Immobilisierung-Ligand-Bindungspartner enthält; und
(c) die Komplexe an den Mikropartikeln durch immunologische Bindung zwischen dem Immobilisierung-Ligand und dem Immobilisierung-Ligand-Bindungspartner immobilisiert werden.

11. Test-Kit zur Durchführung eines PSA-ACT-Assay mit einer Blutprobe, umfassend:
(a) einen ersten Antikörper, einen zweiten Antikörper, Mikropartikel mit einem mittleren Durchmesser von 0,01 bis 100 µm und nachweisbare Verbindungen;
(b) worin der erste Antikörper dazu befähigt ist, immunologisch mit PSA von PSA-ACT gebunden zu werden;
(c) worin der zweite Antikörper dazu befähigt ist, immunologisch mit ACT von PSA-ACT gebunden zu werden; und
(d) worin jeder der ersten und zweiten Antikörper dazu befähigt ist, an den Mikropartikeln immobilisiert oder mit den nachweisbaren Verbindungen markiert zu werden.

12. Test-Kit gemäß Anspruch 11, worin:
(a) die Mikropartikel einen mittleren Durchmesser von 1 bis 50 µm aufweisen.

## Revendications

1. Procédé pour la mise en oeuvre d'une analyse de PSA-ACT en utilisant un échantillon de sang, comprenant les étapes consistant :
(a) à former un mélange comprenant un premier anticorps, un second anticorps, du PSA-ACT, et des microparticules ayant un diamètre moyen compris dans l'intervalle de 0,01 µm (micromètres) à 100 µm (micromètres) ;
(b) le premier anticorps pouvant être lié immunologiquement avec le PSA du PSA-ACT ;
(c) le second anticorps pouvant être lié immunologiquement avec la ACT du PSA-ACT ;
(d) chacun des anticorps consistant en le premier anticorps et le second anticorps pouvant être immobilisé aux microparticules ou marqué avec des composés détectables ;
(e) à former des complexes comprenant le premier anticorps, le second anticorps et le PSA-ACT, dans lesquels (I) le premier anticorps est immunologiquement lié avec le PSA du PSA-ACT, (II) le second anticorps est immunologiquement lié avec la ACT du PSA-ACT et (III) les complexes sont immobilisés aux microparticules et marqués avec les composés détectables ;
(f) à séparer les complexes dans une partie du mélange du reste du mélange ;
(g) à engendrer des signaux en rapport avec les composés détectables ;
(h) à détecter les signaux ; et
(i) à déterminer, sur la base d'une relation proportionnelle entre les signaux détectés et la concentration de PSA-ACT, la quantité de PSA-ACT.

2. Procédé répondant à la définition suivant la revendication 1, dans lequel :
(a) les microparticules ont un diamètre moyen compris dans l'intervalle de 1 µm (micromètres) à 50 µm (micromètres).

3. Procédé répondant à la définition suivant la revendication 1, dans lequel :
(a) la séparation des complexes utilise un liquide de lavage et est effectuée par un analyseur automatisé.

4. Procédé répondant à la définition suivant la revendication 1, dans lequel :
(a) chacun des anticorps consistant en le premier anticorps et le second anticorps est un anticorps monoclonal.

5. Procédé répondant à la définition suivant la revendication 1, dans lequel :
(a) les microparticules sont sous forme de latex, de colloïdes ou de gels hydratables.

6. Procédé répondant à la définition suivant la revendication 5, dans lequel :
(a) les microparticules sont des latex de polymère.

7. Procédé répondant à la définition suivant la revendication 1, dans lequel :
(a) les microparticules sont magnétiquement sensibles.

8. Procédé répondant à la définition suivant la revendication 7, dans lequel :
(a) les microparticules magnétiquement sensibles sont des matrices de polymère non magnétique contenant de l'oxyde de fer.

9. Procédé répondant à la définition suivant la revendication 1, dans lequel :
(a) les composés détectables sont enzymatiques, chimioluminescents, fluorescents ou radio-actifs.

10. Procédé répondant à la définition suivant la revendication 1, dans lequel :
(a) chacun des anticorps consistant en le premier anticorps ou le second anticorps est modifié avec un ligand d'immobilisation ;
(b) les microparticules contiennent, à l'état fixé sur celles-ci, un partenaire de liaison de ligand d'immobilisation ; et
(c) les complexes sont immobilisés aux microparticules par liaison immunologique entre le ligand d'immobilisation et le partenaire de liaison de ligand d'immobilisation.

11. Kit analytique pour la mise en oeuvre d'une analyse de PSA-ACT en utilisant un échantillon de sang, comprenant :
(a) un premier anticorps, un second anticorps, des microparticules ayant un diamètre moyen compris dans l'intervalle de 0,01 µm (micromètres) à 100 µm (micromètres) et des composés détectables ;
(b) le premier anticorps pouvant être lié immunologiquement avec le PSA du PSA-ACT ;
(c) le second anticorps pouvant être lié immunologiquement avec la ACT du PSA-ACT ; et
(d) chacun des anticorps consistant en le premier anticorps et le second anticorps pouvant être immobilisé aux microparticules ou marqué avec des composés détectables.

12. Kit analytique répondant à la définition suivant la revendication 11, dans lequel :
(a) les microparticules ont un diamètre moyen compris dans l'intervalle de 1 µm (micromètres) à 50 µm (micromètres).
